# FASCICULE DE BREVET EUROPEEN

(11) **EP 3 943 399 B1**
(45) Date de publication et mention de la délivrance du brevet: **02.08.2023**
(21) Numéro de dépôt: 21180865.4
(22) Date de dépôt: 22.06.2021
(51) Int. Cl.: B64F 1/36

(54) **SYSTEME ET PROCEDE DE REGULATION DE LA TEMPERATURE DE LA CABINE D'UN AERONEF AU SOL**
SYSTEM UND VERFAHREN ZUM REGULIEREN DER KABINENTEMPERATUR EINES LUFTFAHRZEUGS AM BODEN
SYSTEM AND METHOD FOR CONTROLLING THE TEMPERATURE OF THE CABIN OF AN AIRCRAFT ON THE GROUND

(30) Priorité: 20.07.2020 FR 2007566
(43) Date de publication de la demande: 26.01.2022
(73) Titulaire: Airbus (S.A.S.), 31700 Blagnac (FR); Airbus Operations GmbH, 21129 Hamburg (DE); Airbus Defence and Space GmbH, 82024 Taufkirchen (DE)
(72) Inventeur: CHANTAL, Philippe, 31700 Blagnac (FR); CHABAUD, Eloi, 31700 Blagnac (FR); KRAKOWSKI, Dariusz, 21129 Hamburg (DE); DUHOVNIKOV, Svetoslav, 82024 Taufkirchen (DE); KUBISCH, Martin, 82024 Taufkirchen (DE)
(74) Mandataire: Sarraméa, Claude

(56) Documents cités:
- CN-A- 103 836 767
- CN-B- 102 358 433
- CN-B- 102 622 829
- US-A1- 2012 067 965
- US-A1- 2019 201 564

## Description

La présente demande se rapporte à un système et un procédé de régulation de la température de la cabine d'un aéronef au sol, permettant une désinfection efficace de l'intégralité de la cabine.

De nos jours, malgré certaines précautions sanitaires, des passagers d'un aéronef peuvent être infectés d'un virus transmissible notamment via des surfaces de contact de la cabine. Ainsi, une cabine est potentiellement exposée à des passagers infectés.

Afin d'éviter la propagation des virus, les cabines sont régulièrement désinfectées.

De façon connue, certains virus ne sont plus actifs s'ils sont exposés à une température prédéterminée pendant une durée déterminée.

Afin d'amener une cabine d'un aéronef à la température prédéterminée, et donc de désinfecter la cabine, une unité d'air conditionné (PCA, acronyme de l'expression Anglo-saxonne « Pre-conditioned Air Unit ») au sol est généralement utilisée.

Le document CN 103 836 767 A divulgue un système de commande pour la cabine d'un aéronef au sol. Le système comprend des capteurs, situés dans la cabine de l'avion et configurés pour mesurer en temps réel des données liées à l'environnement de la cabine et liées à la sortie d'un climatiseur au sol. Ces données sont ensuite utilisées pour déterminer la température et l'humidité dans la cabine.

Les mesures des capteurs sont transmises à un système de traitement des données, qui est relié à un contrôleur.

Le document CN 102 622 829 B divulgue un système d'alimentation en énergie et en air conditionné d'aéronef au sol.

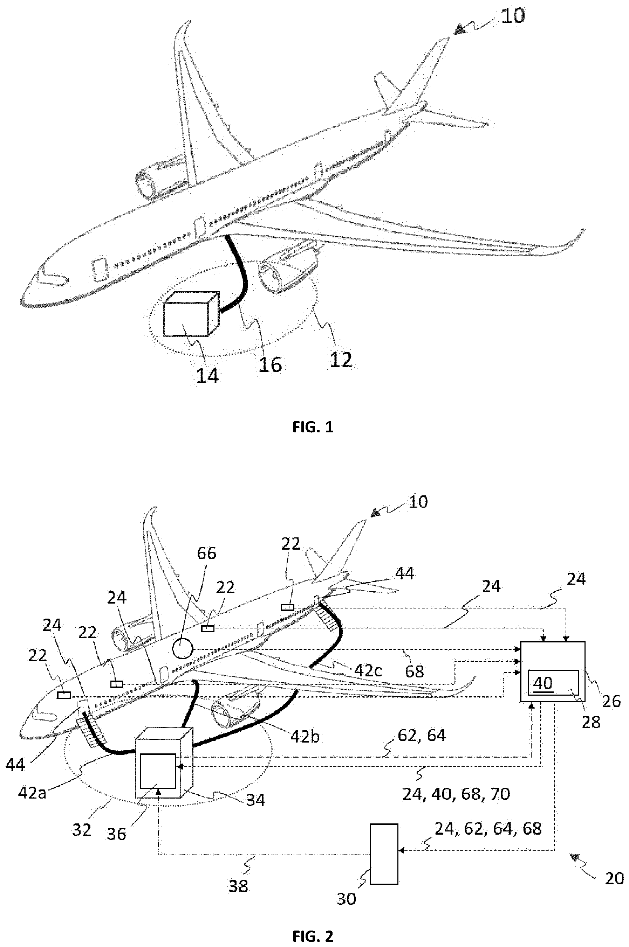

La figure 1 représente un aéronef 10 au sol, sur lequel une unité d'air conditionné 12 externe à l'aéronef 10 est connectée. L'unité d'air conditionné 12 comporte un générateur d'air conditionné 14 qui est fluidiquement connecté, via un conduit d'air 16 flexible, aux canalisations d'air (non visibles sur la figure 1) de l'aéronef 10. Le générateur d'air conditionné 14 est configuré pour distribuer de l'air dans la cabine selon un cycle prédéterminé de régulation de la température de l'air de la cabine pendant une durée déterminée. L'unité d'air conditionné 12 est pilotable manuellement par un utilisateur, qui commande l'activation du générateur d'air conditionné 14 et le lancement du cycle prédéterminé. Un cycle de régulation est définit par une première phase d'augmentation (ou diminution) de la température à partir d'une température initiale, jusqu'à une température prédéterminée, puis par une deuxième phase de maintien de la température prédéterminée pendant une durée déterminée, et enfin par une troisième phase de diminution (ou augmentation) de la température prédéterminée jusqu'à une température finale qui correspond à la température initiale.

Toutefois, avec une telle unité d'air conditionné 12, la température de la cabine n'est pas homogène dans toute la cabine. En effet, certaines zones de la cabine de l'aéronef peuvent, par exemple, ne pas atteindre la température prédéterminée pendant toute la durée déterminée. De ce fait, certaines zones de la cabine peuvent, après un cycle de désinfection, encore contenir un virus à l'état actif.

La présente invention vise à proposer une solution permettant d'optimiser la désinfection d'une cabine d'un aéronef.

A cet effet, l'invention a pour objet un système de régulation de la température de la cabine d'un aéronef au sol.

Selon l'invention, le système comprend :
- une pluralité de capteurs configurés pour être desposés dans différentes zones de la cabine, chaque capteur étant configuré pour acquérir, en temps réel, des données représentatives de la température de la zone de la cabine,
- un serveur informatique configuré pour recevoir, en temps réel, les données de la pluralité de capteurs et comprenant une base de données comportant, pour l'aéronef, un cycle prédéterminé de régulation de la température de l'air de la cabine à une température prédéterminée pendant une durée déterminée,
- une unité d'air conditionné au sol, externe à l'aéronef, comprenant :
   - un générateur d'air conditionné pour générer de l'air conditionné dans la cabine, et
   - un module de pilotage connecté au générateur d'air conditionné et configuré pour recevoir les données du serveur informatique,
- une interface utilisateur connectée au serveur informatique, et configurée pour accéder aux données du serveur informatique et pour transmettre un signal de commande au module de pilotage.

Selon l'invention, le module de pilotage est configuré pour, à réception du signal de commande, piloter, en temps réel, le générateur d'air conditionné, selon le cycle prédéterminé, le cycle prédéterminé étant modulé, en temps réel, en fonction des données du serveur informatique.

Avantageusement, le système selon l'invention permet de contrôler, en temps réel, la température de la cabine d'un aéronef, pendant un cycle prédéterminé de régulation de la température de l'air de la cabine. Le système permet ainsi, en temps réel, de vérifier que, pour chaque zone de la cabine, la température de la cabine atteigne une température prédéterminée, pendant une durée déterminée. Le cycle prédéterminé correspondant à un cycle de désinfection de la cabine, le système permet de vérifier que toutes les zones de la cabine de l'aéronef sont correctement exposées à la température prédéterminée pendant la durée prédéterminée, et donc que toutes les zones de la cabine de l'aéronef sont correctement désinfectées.

Selon un premier mode de réalisation, l'interface utilisateur est un téléphone portable, une tablette ou un ordinateur.

Selon un deuxième mode de réalisation, l'unité d'air conditionné comprend l'interface utilisateur.

Selon une caractéristique, la pluralité de capteurs est configurée pour transmettre les données au serveur informatique par une transmission sans fil. Selon cette caractéristique, le serveur informatique est configuré pour transmettre les données au module de pilotage par une transmission sans fil. Selon cette caractéristique, l'interface utilisateur est connectée au serveur informatique par une connexion sans fil.

Selon une autre caractéristique, le module de pilotage est configuré pour, à réception du signal de commande, envoyer des données relatives à sa localisation au serveur informatique.

Selon une autre caractéristique, l'unité d'air conditionné comprend un réservoir contenant au moins une solution désinfectante connecté au générateur d'air conditionné, et le générateur d'air conditionné est configuré pour générer un mélange comprenant de l'air conditionné et la solution désinfectante.

Selon une autre caractéristique, le module de pilotage comprend des moyens de sécurité comprenant un sous-module de comparaison configuré pour comparer la valeur de chaque donnée du serveur informatique avec un seuil prédéterminé, et un sous-module d'arrêt configuré pour arrêter le pilotage du générateur d'air conditionné lorsque que la valeur d'au moins une donnée du serveur informatique est supérieure au seuil prédéterminé.

Selon une autre caractéristique, l'aéronef est muni d'une information permettant d'identifier de manière unique l'aéronef. Selon cette caractéristique, l'interface utilisateur comporte des moyens configurés pour interagir avec l'information permettant d'identifier de détection d'un identifiant manière unique l'aéronef, et est configurée pour accéder, à détection de l'identifiant de l'aéronef, aux données du serveur informatique pour l'aéronef identifié.

L'invention porte également sur un procédé de régulation de la température de la cabine d'un aéronef au sol, au moyen d'un système de régulation comprenant une pluralité de capteurs disposés dans différentes zones de la cabine, un serveur informatique comprenant une base de données comportant, pour l'aéronef, un cycle prédéterminé de régulation de la température de l'air de la cabine à une température prédéterminée pendant une durée déterminée, une interface utilisateur connectée au serveur informatique, et une unité d'air conditionné au sol, externe à l'aéronef, qui comporte un générateur d'air conditionné et un module de pilotage connecté au générateur d'air conditionné.

Selon l'invention, le procédé comprend les étapes consistant en :
- l'acquisition, en temps réel, par la pluralité de capteurs, de données représentatives de la température des différentes zones de la cabine,
- la réception, en temps réel, des données de la pluralité de capteurs par le serveur informatique,
- l'accès, par l'interface utilisateur, aux données du serveur informatique,
- la transmission, par l'interface utilisateur, d'un signal de commande, au module de pilotage, et
- à réception du signal de commande :
   - la réception, par le module de pilotage, en temps réel, des données du serveur informatique,
   - la modulation du cycle prédéterminé, en temps réel, en fonction des données du serveur informatique,
   - le pilotage, en temps réel, du générateur d'air conditionné, selon le cycle prédéterminé modulé.

Selon une caractéristique, l'aéronef est muni d'une information permettant d'identifier de manière unique l'aéronef, et l'interface utilisateur comporte des moyens configurés pour interagir avec l'information permettant d'identifier de détection d'un identifiant manière unique l'aéronef. Selon cette caractéristique, le procédé comprend, avant l'étape d'accès aux données du serveur informatique, les étapes consistant en :
- l'identification, par les moyens configurés pour interagir avec l'information permettant d'identifier de manière unique l'aéronef, de l'aéronef, au moyen de son information permettant d'identifier de manière unique l'aéronef,
- la transmission, par l'interface utilisateur, de l'information permettant d'identifier de manière unique l'aéronef au module de pilotage.

D'autres caractéristiques et avantages ressortiront de la description de l'invention qui va suivre, description donnée à titre d'exemple uniquement, en regard des dessins annexés parmi lesquels :
La figure 1 est une vue de côté d'un aéronef sur lequel une unité d'air conditionné est connectée, qui illustre un mode de réalisation de l'art antérieur,
La figure 2 est une vue en perspective d'un système de régulation de la température de la cabine d'un aéronef au sol, qui illustre un mode de réalisation de l'invention,
La figure 3 est un graphique illustrant l'évolution de la température dans une cabine d'un aéronef en fonction du temps, un système de régulation qui illustre un mode de réalisation de l'invention étant connecté à l'aéronef,
La figure 4 est une vue d'une interface utilisateur d'un système de régulation de la température de la cabine d'un aéronef au sol, qui illustre un mode de réalisation de l'invention,
La figure 5 est une vue en perspective d'un système de régulation de la température de la cabine d'un aéronef au sol, qui illustre un autre mode de réalisation de l'invention, et
La figure 6 est une vue en perspective d'un système de régulation de la température de la température de la cabine d'une flotte d'aéronef, qui illustre un mode de réalisation de l'invention.

Sur la figure 2, un système 20 de régulation de la température de la cabine d'un aéronef 10 est représenté.

On entend par « cabine de l'aéronef », l'intérieur de l'aéronef, c'est-à-dire l'ensemble composé de la cabine où sont installés les sièges des passagers, du poste de pilotage, des baies avioniques et du cockpit de l'aéronef.

Le système 20 comprend une pluralité de capteurs 22 disposés dans la cabine de l'aéronef 10. La cabine est divisée en zones, et au moins un capteur est disposé dans chaque zone. Des capteurs 22 sont disposés dans une partie inférieure de la cabine (définie par rapport au sol), c'est-à-dire au niveau du plancher de la cabine, comme par exemple sous les sièges, dans une partie supérieure de la cabine (définie par rapport au sol), c'est-à-dire au niveau du plafond de la cabine, comme par exemple au niveau des compartiments à bagages, et dans une partie intermédiaire située entre la partie inférieure et la partie supérieure, c'est-à-dire au niveau des sièges.

Chaque capteur 22 est configuré pour acquérir, en temps réel, des données 24 représentatives de la température de la cabine. Une donnée 24 représentative de la température comprend, par exemple, la température elle-même, le débit de l'air, la pression dans la cabine... Lorsqu'un capteur 22 effectue une mesure, le capteur 22 enregistre également la date et l'heure de la mesure. En particulier, chaque capteur 22 est configuré pour acquérir des données 24 représentatives de la température de la zone de la cabine dans laquelle le capteur 22 est agencé. Ainsi, la cabine de l'aéronef est divisée en zones, et des capteurs 22 permettent d'acquérir la température de chacune de ces zones. Les capteurs 22 sont agencés de sorte à ce que la température dans l'intégralité de la cabine puisse être acquise. Les différences de température entre les différentes zones de la cabine sont ainsi enregistrées par les capteurs 22. En effet, de façon connue, la température de l'air dans la partie supérieure de la cabine est plus élevée que dans la partie inférieure de celle-ci.

Chaque capteur 22 comporte un émetteur (non représentés sur les figures) et est configuré pour transmettre les données 24 acquises, via son émetteur. La transmission des données des capteurs 22 est une transmission sans fil.

Le système 20 comprend également un serveur informatique 26 (également connu sous le nom de « cloud » en terminologie Anglo-saxonne). Le serveur informatique 26 comporte un récepteur (non représenté sur les figures) et est configuré pour recevoir, en temps réel, les données 24 des capteurs 22, via son récepteur.

Le serveur informatique 26 comprend une base de données 28 qui comporte, pour l'aéronef 10, un cycle prédéterminé 40 de régulation de la température de l'air de la cabine à une température prédéterminée pendant une durée déterminée. Ce cycle de régulation correspond à un cycle de désinfection de la cabine de l'aéronef.

Ainsi, pour un aéronef 10 donné, la base de données 28 comprend les données 24 des capteurs 22 installés dans l'aéronef 10 donné, et le cycle prédéterminé 40 lié à l'aéronef 10.

Le système 20 comprend également une interface utilisateur 30 connectée au serveur informatique 26, par une connexion sans fil. L'interface utilisateur 30 est configurée pour accéder automatiquement aux données 24 du serveur informatique 26, et pour les afficher automatiquement à un utilisateur du système 20.

Le serveur informatique 26 comporte donc un émetteur (non représenté sur les figures) et est configuré pour transmettre les données 24 des capteurs 22 à l'interface utilisateur 30, via son émetteur. L'interface utilisateur 30 comporte donc un récepteur (non représenté sur les figures) et est configurée pour recevoir les données 24 des capteurs 22 transmises par le serveur informatique 26, via son récepteur.

Le système 20 comprend également une unité d'air conditionné 32 au sol, externe à l'aéronef 10.

On entend par « air conditionné », un air dont la température est contrôlée pour obtenir la température prédéterminée.

L'unité d'air conditionné 32 comprend un générateur d'air conditionné 34 pour générer de l'air conditionné dans la cabine, et un module de pilotage 36 connecté au générateur d'air conditionné 34.

L'interface utilisateur 30 comporte donc un émetteur (non représenté sur les figures) et est configurée pour, une fois que l'interface utilisateur 30 a accès aux données 24 du serveur informatique 26, transmettre un signal de commande 38 au module de pilotage 36.

Le module de pilotage 36 comporte un récepteur (non représenté sur les figures) et est configuré pour, à réception du signal de commande 38, recevoir, en temps réel, les données du serveur informatique 26, via son récepteur. Les données du serveur informatique 26 comportent les données 24 des capteurs 22 et le cycle prédéterminé 40. La transmission des données du serveur informatique 26 au module de pilotage 36 est une transmission sans fil. Le module de pilotage 36 est configuré pour piloter, en temps réel, le générateur d'air conditionné 34 selon le cycle prédéterminé 40.

En fonction des données 24 des capteurs 22 reçues par le module de pilotage 36, le cycle prédéterminé 40 est modulé, en temps réel. Le module de pilotage 36 pilote donc, en temps réel, le générateur d'air conditionné 34 selon le cycle prédéterminé modulé. Le générateur d'air conditionné 34 génère ainsi de l'air dans la cabine de l'aéronef 10 avec une consigne de température, de débit de l'air conditionné, et de durée prédéterminés, de manière à respecter le cycle prédéterminé modulé.

Le module de pilotage 36 comporte un émetteur (non représenté sur les figures) et est configuré pour transmettre une information relative au cycle prédéterminé modulé 62, en temps réel, au serveur informatique 26, via son émetteur.

Le module de pilotage 36 envoie également des données de localisation au serveur informatique 26, et une indication relative à l'utilisation de l'unité d'air conditionné 34.

Une fois le cycle prédéterminé modulé achevé, le module de pilotage 36 arrête le pilotage du générateur d'air conditionné 34, et le générateur d'air conditionné 34 s'arrête. Le module de pilotage 36 envoie alors au serveur informatique 26 une indication relative à la fin de l'utilisation de l'unité d'air conditionné 34.

A la fin du cycle de régulation, la cabine de l'aéronef 10 est ainsi désinfectée, grâce à la régulation de la température à la température prédéterminée pendant la durée déterminée.

Ce système 20 permet ainsi de générer de l'air dans la cabine d'un l'aéronef, à une température prédéterminée, pendant une durée déterminée, de manière automatique afin de désinfecter la cabine de l'aéronef.

En outre, comme les capteurs 22 sont disposés dans différentes zones de la cabine, et que le cycle de régulation est modulé par les données 24 des capteurs 22, en temps réel, le système 20 permet une désinfection de toutes les zones de la cabine, puisque chaque zone de la cabine a vu sa température être régulée selon le cycle prédéterminé. En effet, avec le système 20, la température de chaque zone de la cabine a été à la température prédéterminée pendant la durée déterminée.

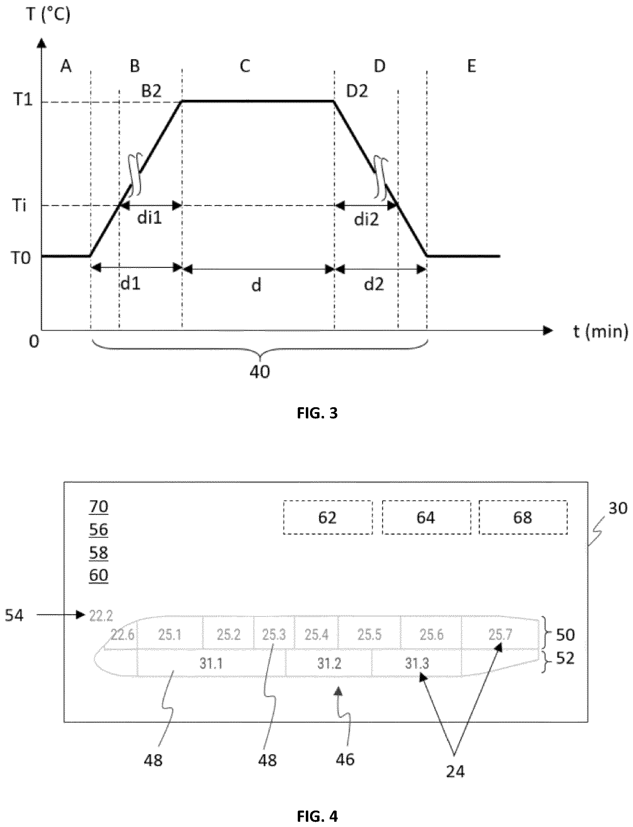

La figure 3 illustre le cycle prédéterminé 40 de régulation de la température de l'air de la cabine à une température prédéterminée pendant une durée déterminée. Le cycle prédéterminé 40 est définit par :
- une première phase B d'augmentation (ou diminution) de la température, à partir d'une température initiale T0, jusqu'à une température prédéterminée T1, pendant une durée déterminée d1 ;
- une deuxième phase C de maintien de la température prédéterminée T1 pendant une durée déterminée d ;
- une troisième phase D de diminution (ou augmentation) de la température prédéterminée T1 jusqu'à une température finale qui correspond à la température initiale T0, pendant une durée déterminée d2.

Si le cycle de régulation requiert un chauffage de l'air de la cabine jusqu'à la température prédéterminée T1 qui est supérieure à la température initiale T0 de la cabine, la première phase B est une augmentation de la température, et la troisième phase D est une diminution de la température. Dans le cas contraire, si le cycle de régulation requiert un refroidissement de l'air de la cabine jusqu'à la température prédéterminée T1 qui est inférieure à la température initiale T0 de la cabine, la première phase B est une diminution de la température, et la troisième phase D est une augmentation de la température.

La phase B correspond à une initialisation de la désinfection de la cabine, la phase C correspond à la désinfection de la cabine, et la phase D correspond à une initialisation de la fin de la désinfection de la cabine.

Avant le cycle prédéterminé (phase A sur la figure 3), la température de la cabine est égale à la température initiale T0, et après le cycle prédéterminé (phase E sur la figure 3), la température de la cabine est égale à la température initiale T0.

La température initiale de la cabine peut être différente de la température initiale T0 définie selon le cycle prédéterminé. Par exemple, la température initiale de la cabine est égale à une température Ti, comprise entre la température initiale T0 du cycle prédéterminé, et la température prédéterminée T1. L'information concernant la température initiale Ti de la cabine est donnée par les données 24 des capteurs 22, qui sont reçues par le module de pilotage 36.

Dans ce cas, le cycle prédéterminé 40 est modulé.

Le cycle prédéterminé modulé est alors définit par une première phase B2 d'augmentation (ou diminution) de la température, à partir de la température initiale Ti, jusqu'à la température prédéterminée T1, pendant une durée déterminée di1; puis par la deuxième phase C et la troisième phase D telles que définies ci-dessus.

En variante, le cycle prédéterminé modulé est définit par une première phase B2 d'augmentation (ou diminution) de la température, à partir de la température initiale Ti, jusqu'à la température prédéterminée T1, pendant une durée déterminée di1; puis par la deuxième phase C telle que définie ci-dessus, et enfin par une troisième phase D2 de diminution (ou augmentation) de la température prédéterminée T1 jusqu'à une température finale qui correspond à la température initiale Ti, pendant une durée déterminée di2.

Ainsi, le module de pilotage 36, à réception des données 24 des capteurs 22 et du cycle prédéterminé 40, module le cycle prédéterminé 40, c'est-à-dire détermine le point de départ et le point d'arrivée du cycle prédéterminé 40 à considérer pour réguler la température de la cabine 10 de l'aéronef.

Selon une configuration, le cycle prédéterminé 40 définit une température prédéterminée T1 comprise entre 56°C et 68°C, et une durée déterminée d comprise entre 15°minutes et 40°minutes. Ainsi, le cycle prédéterminé est un cycle de chauffage de la cabine de l'aéronef. Bien entendu, un cycle de refroidissement (ou climatisation) de la cabine de l'aéronef peut également être mis en oeuvre.

Selon un mode de réalisation représenté sur la figure 2, l'interface utilisateur 30 est un dispositif électronique tel qu'un téléphone portable, une tablette ou un ordinateur. Plus précisément, l'interface utilisateur 30 est une application sur l'un de ces dispositifs électroniques. Ainsi, l'interface utilisateur 30 est agencée à distance de l'unité d'air conditionné 32. La désinfection d'une cabine d'un aéronef est ainsi commandée automatiquement.

La figure 4 représente un exemple d'une interface utilisateur 30, qui est configurée pour afficher un modèle 46 simplifié de la cabine de l'aéronef, sur lequel les zones 48 de la cabine, en partie supérieure 50 et inférieure 52 de la cabine apparaissent. L'interface utilisateur 30 affiche également, en temps réel, les données 24 des capteurs, la date 56 et l'heure 58 des données 24, la température extérieure 54 de l'aéronef, la localisation 60 (données GPS, acronyme de « Géolocalisation Par Satellite ») de l'aéronef, ainsi que le cycle prédéterminé modulé 62 reçu par le serveur informatique 26. Ainsi, un utilisateur a accès, en temps réel, à une indication de la phase du cycle de régulation qui est en cours.

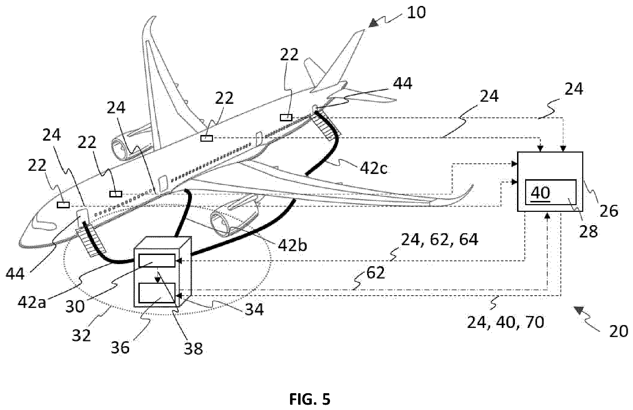

Selon un autre mode de réalisation représenté sur la figure 5, l'unité d'air conditionné 32 comprend l'interface utilisateur 30. L'interface utilisateur 30 est alors une interface homme-machine, directement intégrée dans l'unité d'air conditionné 32. La désinfection d'une cabine d'un aéronef est alors commandée manuellement.

Selon une configuration représentée sur la figure 2, le générateur d'air conditionné 34 comprend plusieurs conduits d'air 42a, 42b, 42c flexibles connectés à l'aéronef 10, et notamment aux portes d'accès 44 de l'aéronef 10. Bien entendu, cette configuration n'est pas limitative, et le générateur d'air conditionné 34 peut comprendre un, deux, ou plus de trois conduits d'air flexibles connectés à l'aéronef 10.

Selon une configuration, l'unité d'air conditionné 32 comprend un réservoir (non représenté sur les figures) contenant au moins une solution désinfectante. Une solution désinfectante comprend au moins un produit désinfectant, c'est-à-dire un produit permettant de désinfecter une surface ou l'environnement au niveau duquel la solution désinfectante est dispersée, i.e. de désactiver les virus présents sur cette surface ou dans l'environnement au niveau duquel la solution désinfectante est dispersée. Le réservoir est connecté à une sortie d'air du générateur d'air conditionné 34. Le générateur d'air conditionné 34 est configuré pour générer un mélange comprenant de l'air conditionné et un ou plusieurs produits désinfectants.

Selon un mode de réalisation, le module de pilotage 36 comprend des premiers moyens sécurité (non représentés sur les figures), qui sont configurés pour comparer la valeur de chaque donnée 24 des capteurs 22 transmises par le serveur informatique 26 avec un premier seuil prédéterminé, et pour arrêter le pilotage du générateur d'air conditionné 34 lorsque que la valeur d'au moins une donnée 24 est supérieure au premier seuil prédéterminé. Plus précisément, les premiers moyens de sécurité comprennent un sous-module de comparaison (non représenté sur les figures) configuré pour comparer la valeur de chaque donnée 24 des capteurs 22 transmises par le serveur informatique 26 avec un premier seuil prédéterminé, et un sous-module d'arrêt (non représenté sur les figures) configuré pour arrêter le pilotage du générateur d'air conditionné 34 lorsque que la valeur d'au moins une donnée 24 est supérieure au premier seuil prédéterminé. Le sous-module de comparaison peut prendre la forme d'un comparateur, et le sous-module d'arrêt peut prendre la forme d'un interrupteur ou d'un actionneur. Le générateur d'air conditionné 34 arrête alors la génération d'air conditionné dans la cabine. Ces premiers moyens de sécurité permettent de protéger les équipements de la cabine de l'aéronef 10 d'une surchauffe. En effet, certains équipements de la cabine, tels que les canalisations d'air, sont conçus pour supporter une température maximale d'environ 70°C. Les premiers moyens de sécurité permettent d'éviter que cette température maximale ne soit atteinte pendant le cycle prédéterminé.

Selon un autre mode de réalisation, le module de pilotage 36 comprend des deuxièmes moyens sécurité (non représentés sur les figures), qui sont configurés pour comparer la valeur de chaque donnée 24 des capteurs 22 transmises par le serveur informatique 26 avec un deuxième seuil prédéterminé, et pour arrêter le pilotage du générateur d'air conditionné 34 lorsque que la valeur d'au moins une donnée 24 est inférieure au deuxième seuil prédéterminé. Plus précisément, les deuxièmes moyens de sécurité comprennent un sous-module de comparaison (non représenté sur les figures), tel qu'un comparateur, configuré pour comparer la valeur de chaque donnée 24 des capteurs 22 transmises par le serveur informatique 26 avec un premier seuil prédéterminé, et un sous-module d'arrêt (non représenté sur les figures), tel qu'un interrupteur ou un actionneur, configuré pour arrêter le pilotage du générateur d'air conditionné 34 lorsque que la valeur d'au moins une donnée 24 est supérieure au premier seuil prédéterminé. Le générateur d'air conditionné 34 arrête alors la génération d'air conditionné dans la cabine. Ces deuxièmes moyens de sécurité permettent de protéger les équipements de la cabine de l'aéronef 10 d'un surrefroidissement. En effet, certains équipements de la cabine peuvent être conçus pour supporter une température minimale, inférieure au deuxième seuil prédéterminé. Les deuxièmes moyens de sécurité permettent d'éviter que cette température minimale ne soit atteinte pendant le cycle prédéterminé.

Lorsqu'un cycle de régulation est interrompu sur activation des moyens de sécurité, une information 64 relative à l'interruption du cycle prédéterminé est transmise, en temps réel, par le module de pilotage 36, au serveur informatique 26. Cette information 64 est ensuite affichée sur l'interface utilisateur 30. Ainsi, l'utilisateur a accès, en temps réel, à l'information relative à l'interruption du cycle de régulation.

Selon un mode de réalisation représenté sur la figure 2, le système 20 comprend un dispositif de sécurité 66 qui comprend des capteurs (non représentés sur les figures) déployés dans plusieurs zones de la cabine et configurés pour acquérir, en temps réel, des données représentatives de la température des différentes zones de la cabine, un comparateur (non représenté sur les figures) configuré pour recevoir, en temps réel, les données acquises par ces capteurs et pour comparer, en temps réel, la valeur de ces données à un seuil prédéterminé, et un émetteur (non représenté sur les figures) configuré pour transmettre, en temps réel, un signal d'arrêt 68 lorsque la valeur d'une donnée d'un de ces capteurs est supérieure au seuil prédéterminé. Le signal d'arrêt 68 est transmis, en temps réel, par le dispositif de sécurité 66 au serveur informatique 26, qui transmet, en temps réel, le signal d'arrêt 68 au module de pilotage 36. A réception du signal d'arrêt 68, le module de pilotage 36 arrête le pilotage du générateur d'air conditionné 34, qui arrête alors la génération d'air conditionné dans la cabine. Le signal d'arrêt 68 est également transmis par le serveur informatique 36 à l'interface utilisateur 30, qui l'affiche. Le dispositif de sécurité 66 est mobile dans les différentes zones de la cabine. Ce dispositif de sécurité 66 est indépendant des moyens de sécurité du module de pilotage 36, autonome et permet une redondance des moyens de sécurité contre le risque de surchauffe de la cabine.

Selon un mode de réalisation, afin de se connecter à l'interface utilisateur 30, un utilisateur fournit un identifiant utilisateur et un mot de passe qui lui sont propre. Des paramètres d'accès aux données du serveur informatique 26 sont associés à l'identifiant utilisateur. Par exemple, un utilisateur peut avoir accès qu'à certaines données de la base de données 28 du serveur informatique 26.

Selon un mode de réalisation, l'aéronef est muni d'un identifiant, tel qu'un QR code (de l'expression Anglo-saxonne « Quick Response code » signifiant code à Réponse Rapide) ou une étiquette NCF (de l'expression Anglo-saxonne « Near Field Communication » signifiant Communication en Champ Proche). L'identifiant peut également être le numéro de l'aéronef. L'identifiant de l'aéronef permet d'identifier ce dernier de manière unique. L'identifiant de l'aéronef correspond à une information permettant d'identifier de manière unique l'aéronef, parmi d'autres aéronefs.

Les capteurs 22 sont configurés pour transmettre les données 24 au serveur informatique 26 avec une indication de l'identifiant de l'aéronef. Le serveur informatique 26 reçoit les données 24 des capteurs, et les enregistre dans la base données 28 avec l'indication de l'identifiant de l'aéronef.

L'interface utilisateur 30 comporte des moyens de détection (non représentés sur les figures) d'un identifiant. Ces moyens de détection peuvent comprendre une caméra, ou un capteur, qui interagit avec un identifiant d'un aéronef, de manière à identifier un aéronef parmi une pluralité d'aéronefs.

Selon une configuration, la détection de l'identifiant de l'aéronef consiste en une activation d'une balise NFC d'un téléphone portable sur lequel l'interface utilisateur 30 est installée, et en la réception de l'étiquette NFC de l'aéronef.

Selon une autre configuration, la détection de l'identifiant de l'aéronef consiste en la lecture du QR code en utilisant un téléphone portable sur lequel l'interface utilisateur 42 est installée.

Avec l'identifiant (QR code ou balise NFC) de l'aéronef, l'interface utilisateur 30 reçoit une donnée de localisation de l'aéronef, qui est affichée (référence 60 sur la figure 4).

Après identification de l'utilisateur, et identification de l'aéronef, l'interface utilisateur 30 est configurée pour accéder automatiquement aux données du serveur informatique 26 uniquement pour l'aéronef identifié, et pour les afficher automatiquement. L'interface utilisateur 30 affiche également l'identifiant 70 de l'aéronef (comme représenté sur la figure 4). L'identification de l'aéronef permet de renforcer la sécurité du système 20, en ne permettant l'accès aux données 24 des capteurs qu'après une identification de l'utilisateur, et que pour l'aéronef identifié.

Après identification de l'utilisateur, et identification de l'aéronef, l'interface utilisateur 30 est configurée pour transmettre, avec le signal de commande 38, l'identifiant 70 de l'aéronef, au module de pilotage 36.

Le module de pilotage 36 est configuré pour recevoir, en temps réel, uniquement les données du serveur informatique 26 liées à l'identifiant 70 de l'aéronef.

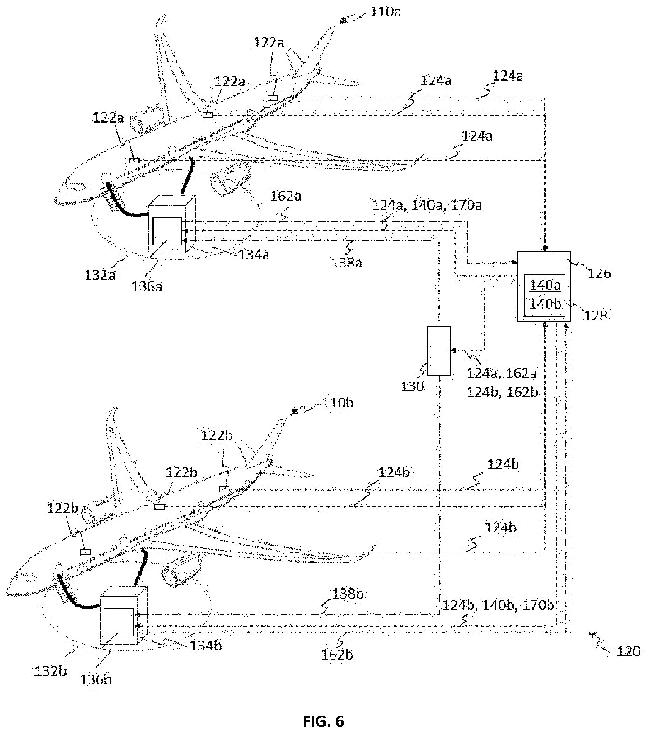

La figure 6 représente un système 120 de régulation de la température de la cabine d'une flotte d'aéronefs 110a, 110b au sol. Une flotte d'aéronefs comporte une pluralité d'aéronefs, de même type ou de types différents. Le type d'un aéronef est défini par le modèle de l'aéronef, c'est-à-dire le nombre de passagers que l'aéronef peut accueillir et le nombre de kilomètres que l'aéronef peut parcourir en autonomie. Par exemple, sur la figure 6, l'aéronef 110a est d'un premier type et l'aéronef 110b est d'un deuxième type différent du premier type.

Chaque aéronef 110a, 110b est muni d'un identifiant 170a, 170b qui lui est propre.

Chaque aéronef 110a, 110b comporte une pluralité de capteurs 122a, 122b installés dans différentes zones de sa cabine. Les capteurs 122a, 122b ont le même fonctionnement que les capteurs 22 décrits précédemment.

Une unité d'air conditionné 132a, 132b, externe à l'aéronef 110a, 110b, est connectée à chaque aéronef 110a, 110b. Chaque unité d'air conditionné 132a, 132b comporte un générateur d'air conditionné 134a, 134b connecté à un module de pilotage 136a, 136b. Chaque unité d'air conditionné 132a, 132b a le même fonctionnement que l'unité d'air conditionné 32 décrite précédemment.

Les capteurs 122a, 122b de chaque aéronef 110a, 110b sont configurés pour acquérir, en temps réel, et transmettre des données 124a, 124b à un serveur informatique 126, avec une indication de son identifiant 170a, 170b. Un unique serveur informatique 126 reçoit, en temps réel, les données 124a, 124b des capteurs 122a, 122b des aéronefs 110a, 110b.

Le serveur informatique 126 comprend une base de données 128 comportant, pour chaque aéronef 110a, 110b, un cycle prédéterminé 140a, 140b de régulation de la température de l'air de la cabine pendant une durée déterminée. Le cycle prédéterminé 140a, 140b dépend du type de l'aéronef 110a, 110b. Comme les aéronefs 110a, 110b sont de types différents, le cycle prédéterminé 140a pour l'aéronef 110a est différent du cycle prédéterminé 140b pour l'aéronef 110b.

Ainsi, pour chaque aéronef 110a, 110b, la base de données 128 comprend les données 124a, 124b des capteurs 122a, 122b et le cycle prédéterminé 140ab 140b.

Le système 120 comporte également une interface utilisateur 130 connectée au serveur informatique 126. L'interface utilisateur 130 peut être unique, ou le système 120 peut comporter plusieurs interfaces utilisateurs 130. Sur la figure 6, l'interface utilisateur 130 est une application d'un téléphone portable, d'une tablette ou d'un ordinateur. Un utilisateur se connecte à l'interface utilisateur 130, via son identifiant utilisateur et son mot de passe.

Ensuite, l'aéronef 110a, 110b pour lequel l'utilisateur souhaite réguler la température de la cabine, par exemple l'aéronef 110a, est identifié. Pour cela, l'identifiant 170a de l'aéronef 110a est détecté, par exemple par lecture du QR code de l'aéronef 110a, ou par activation de la balise NFC de l'aéronef 110a, par les moyens de détection de l'interface utilisateur 130.

Suite à l'identification de l'aéronef 110a, l'interface utilisateur 130 est configurée pour accéder aux données 124a des capteurs 122a de l'aéronef 110a, qui sont enregistrées sur le serveur informatique 126. L'interface utilisateur 130 ne permet pas l'accès aux autres données de la base de données 128. L'accès n'est donné à l'utilisateur qu'aux données 124a qui sont liées à l'identifiant 170a de l'aéronef 110a. L'interface utilisateur 130 est configurée pour afficher automatiquement ces données 124a, ainsi que l'identifiant 170a de l'aéronef 110a.

L'utilisateur, sur la base de l'identifiant 170a de l'aéronef 110a, a accès, via l'interface utilisateur 130, à la localisation de l'aéronef 110a. L'utilisateur a également accès, via l'interface utilisateur 130 et le serveur informatique 126, aux données de localisation des unités d'air conditionné de l'aéroport sur lequel stationné l'aéronef 110a au sol, et à une indication relative à leur utilisation actuelle. Les données de localisation des unités d'air conditionné correspondent à leur dernière localisation connue (donnée de localisation envoyée au serveur informatique 126 lors d'une utilisation précédente). L'utilisateur peut alors sélectionner l'unité d'air conditionné, et donc le module de pilotage, à laquelle l'interface utilisateur 130 va transmettre un signal de commande 138a, en fonction des données de localisation des unités d'air conditionné et de leur disponibilité. Par exemple, l'utilisateur sélectionne l'unité d'air conditionné 134a disponible, c'est-à-dire non actuellement en cours d'utilisation, qui est la plus proche de l'aéronef 110a.

Afin de désinfecter la cabine de l'aéronef 110a, l'utilisateur commande, via l'interface utilisateur 130, la transmission d'un signal de commande 138a au module de pilotage 136a.

A réception du signal de commande 138a, le module de pilotage 136a reçoit, en temps réel, les données du serveur informatique 126. Les données du serveur informatique 126 comportent les données 124a des capteurs 122a et le cycle prédéterminé 140a. Le module de pilotage 136a pilote, en temps réel, le générateur d'air conditionné 134a selon le cycle prédéterminé 140a, ou selon le cycle prédéterminé modulé 162a en fonction des données 124a. Le générateur d'air conditionné 134a génère ainsi de l'air dans la cabine de l'aéronef 110a avec une consigne de température, de débit de l'air conditionné, et de durée prédéterminés, de manière à respecter le cycle prédéterminé modulé 162a.

Une fois le signal de commande 138a transmis au module de pilotage 136a, l'utilisateur, via l'interface utilisateur 130, identifie l'aéronef 110b pour lequel l'utilisateur souhaite réguler la température de la cabine. L'identification du deuxième aéronef 110b n'interrompt pas le cycle prédéterminé en cours pour le premier aéronef 110a.

Suite à l'identification de l'aéronef 110b, l'interface utilisateur 130 est configurée pour accéder aux données 124b des capteurs 122b de l'aéronef 110b, qui sont enregistrées sur le serveur informatique 126. L'interface utilisateur 130 affiche automatiquement ces données 124b, ainsi que l'identifiant 170b de l'aéronef 110b.

L'utilisateur, sur la base de l'identifiant 170b de l'aéronef 110b, a accès, via l'interface utilisateur 130, à la localisation de l'aéronef 110b, ainsi qu'aux données de localisation des unités d'air conditionné de l'aéroport sur lequel stationné l'aéronef 110b au sol, et à une indication relative à leur utilisation actuelle. L'utilisateur peut alors sélectionner l'unité d'air conditionné à laquelle l'interface utilisateur 130 va transmettre un signal de commande 138b, via son module de pilotage, en fonction des données de localisation des unités d'air conditionné et de leur disponibilité. Par exemple, l'utilisateur sélectionne l'unité d'air conditionné 134b disponible qui est la plus proche de l'aéronef 110b.

Afin de désinfecter la cabine de l'aéronef 110b, l'utilisateur commande, via l'interface utilisateur 130, la transmission d'un signal de commande 138b au module 136b.

A réception du signal de commande 138b, le module de pilotage 136b reçoit, en temps réel, les données du serveur informatique 126. Le module de pilotage 136b pilote, en temps réel, le générateur d'air conditionné 134b selon le cycle prédéterminé 140b, ou selon le cycle prédéterminé modulé 162b en fonction des données 124b. Le générateur d'air conditionné 134b génère ainsi de l'air dans la cabine de l'aéronef 110b avec une consigne de température, de débit de l'air conditionné, et de durée prédéterminés, de manière à respecter le cycle prédéterminé modulé 162b.

Pour chaque aéronef 110a, 110b, une fois le cycle prédéterminé achevé, le module de pilotage 136a, 136b arrête le pilotage du générateur d'air conditionné 134a, 134b, et le générateur d'air conditionné 134a, 134b s'arrête. La cabine de chaque aéronef 110a, 110b est alors désinfectée.

A tout moment, l'utilisateur peut, via l'interface utilisateur 130, arrêter la désinfection d'un aéronef, en transmettant un signal d'arrêt au module de pilotage 136a, 136b.

Un utilisateur peut ainsi, via une même interface utilisateur 130, réguler, en temps réel, et de manière automatique, la température de la cabine de plusieurs aéronefs 110a, 110b d'une flotte.

En particulier, l'utilisateur utilise un produit de programme d'ordinateur comportant un ensemble d'instructions de code de programme qui, lorsqu'elles sont exécutées par un processeur (non représenté sur les figures), configurent le processeur pour mettre en oeuvre un procédé de régulation de la température de la cabine d'un aéronef au sol tel que décrit précédemment.

## Revendications

1. Système (20) de régulation de la température de la cabine d'un aéronef (10) au sol, comprenant:
- une pluralité de capteurs (22) configurés pour être disposés dans différentes zones (48) de la cabine, chaque capteur (22) étant configuré pour acquérir, en temps réel, des données (24) représentatives de la température de la zone (48) de la cabine,
- un serveur informatique (26) configuré pour recevoir, en temps réel, les données (24) de la pluralité de capteurs (22) et comprenant une base de données (28) comportant, pour l'aéronef (10), un cycle prédéterminé (40) de régulation de la température de l'air de la cabine à une température prédéterminée pendant une durée déterminée,
- une unité d'air conditionné (32) au sol, externe à l'aéronef (10), comprenant :
• un générateur d'air conditionné (34) pour générer de l'air conditionné dans la cabine, et
• un module de pilotage (36) connecté au générateur d'air conditionné (34) et configuré pour recevoir les données (24) du serveur informatique (26),
- une interface utilisateur (30) connectée au serveur informatique (26), et configurée pour accéder aux données (24, 40) du serveur informatique (26) et pour transmettre un signal de commande (38) au module de pilotage (36), où le module de pilotage (36) est configuré pour, à réception du signal de commande (38), piloter, en temps réel, le générateur d'air conditionné (34), selon le cycle prédéterminé (40), le cycle prédéterminé (40) étant modulé, en temps réel, en fonction des données (24) du serveur informatique (26).

2. Système (20) selon la revendication 1, **caractérisé en ce que** l'interface utilisateur (30) est un téléphone portable, une tablette ou un ordinateur.

3. Système (20) selon la revendication 1, **caractérisé en ce que** l'unité d'air conditionné (32) comprend l'interface utilisateur (30).

4. Système (20) selon l'une des revendications 1 à 3, **caractérisé en ce que** le module de pilotage (36) est configuré pour, à réception du signal de commande (38), envoyer des données relatives à sa localisation au serveur informatique (26).

5. Système (20) selon l'une des revendications 1 à 4, **caractérisé en ce que** l'unité d'air conditionné (32) comprend un réservoir contenant au moins une solution désinfectante connecté au générateur d'air conditionné (32), et **en ce que** le générateur d'air conditionné (32) est configuré pour générer un mélange comprenant de l'air conditionné et la solution désinfectante.

6. Système (20) selon l'une des revendications 1 à 5, **caractérisé en ce que** le module de pilotage (36) comprend des moyens de sécurité comprenant un sous-module de comparaison configuré pour comparer la valeur de chaque donnée (24) du serveur informatique (26) avec un seuil prédéterminé, et un sous-module d'arrêt configuré pour arrêter le pilotage du générateur d'air conditionné (34) lorsque que la valeur d'au moins une donnée (24) du serveur informatique (26) est supérieure au seuil prédéterminé.

7. Système selon l'une des revendications 1 à 6, dans lequel l'aéronef (10) est muni d'une information (70) permettant d'identifier de manière unique l'aéronef, le système (20) étant **caractérisé en ce que** l'interface utilisateur (30) comporte des moyens configurés pour interagir avec l'information permettant d'identifier de manière unique l'aéronef, et est configurée pour accéder, à détection de ladite information (70) permettant d'identifier de manière unique l'aéronef (10), aux données (24) du serveur informatique (26) pour l'aéronef identifié.

8. Procédé de régulation de la température de la cabine d'un aéronef (10) au sol, au moyen d'un système (20) de régulation comprenant une pluralité de capteurs (22) disposés dans différentes zones (48) de la cabine, un serveur informatique (26) comprenant une base de données (28) comportant, pour l'aéronef, un cycle prédéterminé (40) de régulation de la température de l'air de la cabine à une température prédéterminée pendant une durée déterminée, une interface utilisateur (30) connectée au serveur informatique (26), et une unité d'air conditionné (32) au sol, externe à l'aéronef (10), qui comporte un générateur d'air conditionné (34) et un module de pilotage (36) connecté au générateur d'air conditionné (34), **caractérisé en ce que** le procédé comprend les étapes consistant en :
- l'acquisition, en temps réel, par la pluralité de capteurs (22), de données (24) représentatives de la température des différentes zones (48) de la cabine,
- la réception, en temps réel, des données (24) de la pluralité de capteurs (22) par le serveur informatique (26),
- l'accès, par l'interface utilisateur (30), aux données (24) du serveur informatique (26),
- la transmission, par l'interface utilisateur (30), d'un signal de commande (38), au module de pilotage (36), et
- à réception du signal de commande (38) :
• la réception, par le module de pilotage (36), en temps réel, des données (24, 40) du serveur informatique (26),
• la modulation du cycle prédéterminé (40), en temps réel, en fonction des données (24) du serveur informatique (26),
• le pilotage, en temps réel, du générateur d'air conditionné (34), selon le cycle prédéterminé modulé (62).

9. Procédé selon la revendication 8, l'aéronef (10) étant muni d'une information (70) permettant d'identifier de manière unique l'aéronef, et l'interface utilisateur (30) comportant des moyens configurés pour interagir avec l'information permettant d'identifier de manière unique l'aéronef, **caractérisé en ce que** le procédé comprend, avant l'étape d'accès aux données (24, 40) du serveur informatique (26), les étapes consistant en :
- l'identification, par les moyens configurés pour interagir avec l'information permettant d'identifier de manière unique l'aéronef, de l'aéronef (10), au moyen de son information (70) permettant d'identifier de manière unique l'aéronef,
- la transmission, par l'interface utilisateur (30), de l'information (70) permettant d'identifier de manière unique l'aéronef (10) au module de pilotage (36).

## Patentansprüche

1. System (20) zum Regulieren der Kabinentemperatur eines Luftfahrzeugs (10) am Boden, das umfasst:
- eine Vielzahl von Sensoren (22), die dazu konfiguriert sind, in verschiedenen Zonen (48) der Kabine angeordnet zu werden, wobei jeder Sensor (22) dazu konfiguriert ist, Daten (24), die für die Temperatur der Zone (48) der Kabine repräsentativ sind, in Echtzeit zu erfassen,
- einen Computerserver (26), der dazu konfiguriert ist, die Daten (24) der Vielzahl von Sensoren (22) in Echtzeit zu empfangen, und eine Datenbank (28) umfasst, die für das Luftfahrzeug (10) einen vorbestimmten Zyklus (40) zum Regulieren der Temperatur der Kabinenluft auf eine vorbestimmte Temperatur während einer bestimmten Dauer enthält,
- eine Klimaanlageneinheit (32) am Boden außerhalb des Luftfahrzeugs (10), die umfasst:
• einen Klimagenerator (34) zum Erzeugen der klimatisierten Luft in der Kabine und
• ein Steuermodul (36), das mit dem Klimagenerator (34) verbunden ist und dazu konfiguriert ist, die Daten (24) des Computerservers (26) zu empfangen,
- eine Benutzerschnittstelle (30), die mit dem Computerserver (26) verbunden ist und dazu konfiguriert ist, auf die Daten (24, 40) des Computerservers (26) zuzugreifen und ein Befehlssignal (38) an das Steuermodul (36) zu senden,
wobei das Steuermodul (36) dazu konfiguriert ist, um bei Empfang des Befehlssignals (38) den Klimagenerator (34) in Echtzeit gemäß dem vorbestimmten Zyklus (40) zu steuern, wobei der vorbestimmte Zyklus (40) in Echtzeit in Abhängigkeit von den Daten (24) des Computerservers (26) moduliert wird.

2. System (20) nach Anspruch 1, **dadurch gekennzeichnet, dass** die Benutzerschnittstelle (30) ein Smartphone, ein Tablet oder ein Computer ist.

3. System (20) nach Anspruch 1, **dadurch gekennzeichnet, dass** die Klimaanlageneinheit (32) die Benutzerschnittstelle (30) umfasst.

4. System (20) nach einem der Ansprüche 1 bis 3, **dadurch gekennzeichnet, dass** das Steuermodul (36) dazu konfiguriert ist, bei Empfang des Befehlssignals (38) Daten über seinen Standort zum Computerserver (26) zu senden.

5. System (20) nach einem der Ansprüche 1 bis 4, **dadurch gekennzeichnet, dass** die Klimaanlageneinheit (32) einen Tank umfasst, der mindestens eine Desinfektionslösung enthält und mit dem Klimagenerator (32) verbunden ist, und dass der Klimagenerator (32) dazu konfiguriert ist, eine Mischung zu erzeugen, die die klimatisierte Luft und die Desinfektionslösung umfasst.

6. System (20) nach einem der Ansprüche 1 bis 5, **dadurch gekennzeichnet, dass** das Steuermodul (36) Sicherheitsmittel umfasst, die ein Vergleichssubmodul, das dazu konfiguriert ist, den Wert jeder Dateneinheit (24) des Computerservers (26) mit einem vorbestimmten Schwellenwert zu vergleichen, und ein Anhaltsubmodul umfasst, das dazu konfiguriert ist, die Steuerung des Klimagenerators (34) anzuhalten, wenn der Wert mindestens einer Dateneinheit (24) des Computerservers (26) über dem vorgegebenen Schwellenwert liegt.

7. System nach einem der Ansprüche 1 bis 6, wobei das Luftfahrzeug (10) mit einer Information (70) versehen ist, die ein eindeutiges Identifizieren des Luftfahrzeugs ermöglicht, wobei das System (20) **dadurch gekennzeichnet ist, dass** die Benutzerschnittstelle (30) Mittel enthält, die dazu konfiguriert sind, mit der Information, die ein eindeutiges Identifizieren des Luftfahrzeugs ermöglicht, zu interagieren, und dazu konfiguriert ist, bei Erkennung der Information (70), die ein eindeutiges Identifizieren des Luftfahrzeugs (10) ermöglicht, auf die Daten (24) des Computerservers (26) für das identifizierte Luftfahrzeug zuzugreifen.

8. Verfahren zum Regulieren der Kabinentemperatur eines Luftfahrzeugs (10) am Boden mittels eines Systems (20) zum Regulieren, umfassend eine Vielzahl von Sensoren (22), die in verschiedenen Zonen (48) der Kabine angeordnet sind, einen Computerserver (26), der eine Datenbank (28) umfasst, die für das Luftfahrzeug einen vorbestimmten Zyklus (40) zum Regulieren der Temperatur der Kabinenluft auf eine vorbestimmte Temperatur während einer bestimmten Dauer enthält, eine Benutzerschnittstelle (30), die mit dem Computerserver (26) verbunden ist, und eine Klimaanlageneinheit (32) am Boden außerhalb des Luftfahrzeugs (10), die einen Klimagenerator (34) und ein mit dem Klimagenerator (34) verbundenes Steuermodul (36) enthält, **dadurch gekennzeichnet, dass** das Verfahren Schritte umfasst, die bestehen in:
- der Erfassung von Daten (24), die für die Temperatur der verschiedenen Zonen (48) der Kabine repräsentativ sind, durch die Vielzahl von Sensoren (22) in Echtzeit,
- dem Empfang der Daten (24) von der Vielzahl von Sensoren (22) durch den Computerserver (26) in Echtzeit,
- dem Zugriff auf die Daten (24) des Computerservers (26) durch die Benutzerschnittstelle (30),
- der Übertragung eines Befehlssignals (38) an das Steuermodul (36) durch die Benutzerschnittstelle (30) und
- bei Empfang des Befehlssignals (38):
• dem Empfang der Daten (24, 40) des Computerservers (26) in Echtzeit durch das Steuermodul (36),
• der Modulation des vorbestimmten Zyklus (40) in Echtzeit in Abhängigkeit von den Daten (24) des Computerservers (26),
• der Steuerung des Klimagenerators (34) in Echtzeit entsprechend dem modulierten vorbestimmten Zyklus (62).

9. Verfahren nach Anspruch 8, wobei das Luftfahrzeug (10) mit einer Information (70) versehen ist, die ein eindeutiges Identifizieren des Luftfahrzeugs ermöglicht, und die Benutzerschnittstelle (30) Mittel umfasst, die dazu konfiguriert sind, mit der Information, die ein eindeutiges Identifizieren des Luftfahrzeugs ermöglicht, zu interagieren, **dadurch gekennzeichnet, dass** das Verfahren vor dem Schritt des Zugreifens auf die Daten (24, 40) des Computerservers (26) die Schritte umfasst, die bestehen in:
- der Identifizierung des Luftfahrzeugs (10) durch die Mittel, die dazu konfiguriert sind, mit der Information, die ein eindeutiges Identifizieren des Luftfahrzeugs ermöglicht, zu interagieren, mittels seiner Information (70), die ein eindeutiges Identifizieren des Luftfahrzeugs ermöglicht,
- der Übertragung der Information (70), die ein eindeutiges Identifizieren des Luftfahrzeugs (10) ermöglicht, durch die Benutzerschnittstelle (30) an das Steuermodul (36).

## Claims

1. System (20) for regulating the temperature of the cabin of an aircraft (10) when on the ground, comprising:
- a plurality of sensors (22) configured to be arranged in various zones (48) of the cabin, each sensor (22) being configured to acquire, in real time, data (24) representative of the temperature of the zone (48) of the cabin,
- a computer server (26) configured to receive, in real time, the data (24) from the plurality of sensors (22) and comprising a database (28) containing, for the aircraft (10), a predetermined cycle (40) of regulation of the temperature of the air of the cabin to a predetermined temperature during a given duration,
- a ground pre-conditioned air unit (32), external to the aircraft (10), comprising:
• a pre-conditioned air generator (34) for generating pre-conditioned air in the cabin, and
• a control module (36) connected to the pre-conditioned air generator (34) and configured to receive the data (24) from the computer server (26),
- a user interface (30) connected to the computer server (26), and configured to access the data (24, 40) from the computer server (26) and to transmit a control signal (38) to the control module (36),
wherein the control module (36) is configured, upon receiving the control signal (38), to control, in real time, the pre-conditioned air generator (34), according to the predetermined cycle (40), the predetermined cycle (40) being modulated, in real time, depending on the data (24) from the computer server (26).

2. System (20) according to Claim 1, **characterized in that** the user interface (30) is a portable telephone, a tablet or a computer.

3. System (20) according to Claim 1, **characterized in that** the pre-conditioned air unit (32) comprises the user interface (30).

4. System (20) according to one of Claims 1 to 3, **characterized in that** the control module (36) is configured, upon receiving the control signal (38), to send data relating to its location to the computer server (26) .

5. System (20) according to one of Claims 1 to 4, **characterized in that** the pre-conditioned air unit (32) comprises a reservoir containing at least one disinfectant solution connected to the pre-conditioned air generator (32), and **in that** the pre-conditioned air generator (32) is configured to generate a mixture comprising pre-conditioned air and the disinfectant solution.

6. System (20) according to one of Claims 1 to 5, **characterized in that** the control module (36) comprises safety means comprising a comparison submodule that is configured to compare the value of each data item (24) from the computer server (26) with a predetermined threshold, and a stopping submodule that is configured to stop the control of the pre-conditioned air generator (34) when the value of at least one data item (24) from the computer server (26) is above the predetermined threshold.

7. System according to one of Claims 1 to 6, wherein the aircraft (10) is provided with information (70) for uniquely identifying the aircraft, the system (20) being **characterized in that** the user interface (30) comprises means configured to interact with the information for uniquely identifying the aircraft and is configured, upon detecting said information (70) for uniquely identifying the aircraft (10), to access the data (24) of the computer server (26) for the identified aircraft.

8. Method for regulating the temperature of the cabin of an aircraft (10) when on the ground, using a regulation system (20) comprising a plurality of sensors (22) arranged in various zones (48) of the cabin, a computer server (26) comprising a database (28) containing, for the aircraft, a predetermined cycle (40) of regulation of the temperature of the air of the cabin to a predetermined temperature during a given duration, a user interface (30) connected to the computer server (26), and a ground pre-conditioned air unit (32), external to the aircraft (10), which comprises a pre-conditioned air generator (34) and a control module (36) connected to the pre-conditioned air generator (34), **characterized in that** the method comprises the following steps:
- acquiring, in real time and by means of the plurality of sensors (22), data (24) representative of the temperature of the various zones (48) of the cabin,
- receiving, in real time, the data (24) from the plurality of sensors (22) by means of the computer server (26),
- accessing, via the user interface (30), the data (24) from the computer server (26),
- transmitting, via the user interface (30), a control signal (38), to the control module (36), and
- upon receiving the control signal (38):
• receiving, by the control module (36) and in real time, the data (24, 40) from the computer server (26),
• modulating, in real time, the predetermined cycle (40) depending on the data (24) from the computer server (26),
• controlling, in real time, the pre-conditioned air generator (34), according to the modulated predetermined cycle (62).

9. Method according to Claim 8, the aircraft (10) being provided with information (70) for uniquely identifying the aircraft, and the user interface (30) comprising means configured to interact with the information for uniquely identifying the aircraft, **characterized in that** the method comprises, prior to the step of accessing the data (24, 40) of the computer server (26), the steps of:
- using the means configured to interact with the information for uniquely identifying the aircraft to identify the aircraft (10) by means of its information (70) for uniquely identifying the aircraft,
- transmitting, via the user interface (30), the information (70) for uniquely identifying the aircraft (10) to the control module (36).
